(19) Europäisches Patentamt
European Patent Office
Office européen des brevets

(11) Numéro de publication: **0 157 762**
**B1**

(12) # FASCICULE DE BREVET EUROPEEN

(45) Date de publication du fascicule du brevet: **08.08.90**

(21) Numéro de dépôt: **83902952.7**

(22) Date de dépôt: **19.09.83**

(86) Numéro de dépôt international: **PCT/FR83/00185**

(87) Numéro de publication internationale: **WO 85/01288 28.03.85 Gazette 85/08**

(51) Int. Cl.$^5$: **C 07 D 211/26,** C 07 D 401/00, C 07 D 413/00, C 07 D 405/06, A 61 K 31/445

(54) **NOUVELLES PIPERIDINOGUANIDINES SUBSTITUEES, LEUR PROCEDE DE PREPARATION ET LES COMPOSITIONS PHARMACEUTIQUES EN RENFERMANT.**

(43) Date de publication de la demande:
**16.10.85 Bulletin 85/42**

(45) Mention de la délivrance du brevet:
**08.08.90 Bulletin 90/32**

(84) Etats contractants désignés:
**AT BE CH DE FR GB LI LU NL SE**

(56) Documents cités:
**EP-A-0 035 374**
**GB-A-1 459 506**
**GB-A-2 034 305**

(73) Titulaire: **BOUCHARA S.A.**
**47, rue de Bretagne**
**F-75003 Paris (FR)**

(72) Inventeur: **CORNU, Pierre, Jean**
**100, avenue Kléber**
**F-75116 Paris (FR)**
Inventeur: **PERRIN, Claude**
**5, rue de l'Avenir**
**F-91400 Orsay (FR)**
Inventeur: **DUMAITRE, Bernard**
**24, rue Chemin Vert**
**F-93000 Bobigny (FR)**
Inventeur: **STREICHENBERGER, Gilles**
**30, bld du Château**
**F-92200 Neuilly-sur-Seine (FR)**

(74) Mandataire: **Burtin, Jean-François**
**68, Rue Marjolin**
**F-92300 Levallois Perret (FR)**

**Le dossier contient des informations techniques présentées postérieurement au dépôt de la demande et ne figurant pas dans le présent fascicule.**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**EP 0 157 762 B1**

**Description**

L'invention a pour objet de nouvelles pipéridinoguanidines substituées, leur procédé de préparation ainsi que les compositions pharmaceutiques en renfermant.

L'invention a plus particulièrement pour objet de nouvelles pipéridinoguanidines substituées à l'azote imidique par un radical carboxamide.

Elle a spécifiquement pour objet des (phényloxoalcoyl) pipéridinoguanidines de formule générale I

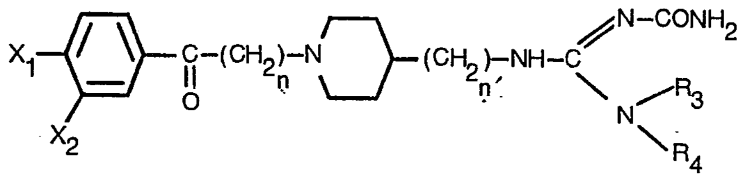

(I)

dans laquelle $X_1$ et $X_2$, identiques ou différents, représentent de l'hydrogène, un radical alcoyle en $C_1$—$C_6$, un radical alcoxy en $C_1$—$C_6$, un halogène ou un radical trifluorométhyle, $R_3$ représente un radical alcoyle ayant de 1 à 6 atomes de Carbone, $R_4$ représente de l'hydrogène, un radical alcoyle ayant de 1 à 6 atomes de carbone ou le reste acyle d'un acide organique carboxylique ayant de 1 à 12 atomes de carbone, ou bien $R_3$ et $R_4$ forment ensemble le radical alcoylène d'un hétérocycle azoté choisi dans le groupe constitué par un radical pyrolidinyl, pipéridinyl, hexaméthylène imino, morpholyl, tétrahydrothiazinyl, hexahydropyrimidinyl, hexahydropyrazinyl, pyrazolidinyl ou imidazolidinyl,

n est égal à 1, 2 ou 3
n' est égal à 0 ou 1

ainsi que les composés suivants: 1-[(4.p-fluorophenyl 4-oxo)-1-butyl]4-[(N-carboxamido N'-allyl) guanidinylmethyl]piperidine, 1-[4-(p fluorophenyl 4-oxo)butyl-1] 4[-(N-carboxamido N'-cydohexyl) guanidinylmethyl]piperidine and 1-[(4-fluorophenyl 4-oxo)butyl-1] 4-(N-Carboxamido N'-allyl-guanidinyl)piperidine sous forme racémique ou sous forme optiquement — active.

La formule générale I représente une des structures possibles des carboxamidoguanidines. En milieu acide l'un quelconque des azotes de la fonction guanidine peut être protoné. Il en résulte que les composés, selon l'invention, peuvent exister sous les deux formes tautomères imino-carboxamidoamine et (amino-carboxamido) imine.

En outre, le groupe carboxamide peut se trouver d'un côté ou de l'autre du plan déterminé par la double liaison —C = N. Il en résulte une possibilité d'isomérie *syn* et *anti.*

Les formes tautomères et les formes isoméres font, partie de l'invention.

L'invention se rapporte aussi aux sels d'addition avec un acide minéral ou organique, de préférence un acide thérapeutiquement compatible, d'un composé de formule générale I.

L'invention se rapporte encore aux formes optiquement active des composés de formule générale I ainsi qu'aux diastéréoisomères des composés de formule générale I.

Parmi les sels d'addition des composés de formule générale I, on pourra citer plus particulièrement les chlorhydrates, bromhydrates, sulfates, nitrates, phosphates, thiosulfates, les formiates, acétates, maléates fumarates, benzoates, dichloro 2,6-benzoates, citrates, tartrates, (méthoxy salicylates), 3,4,5-triméthoxy benzoates, les vanillates, les O-carbéthoxy syringoates, les naphtoates, les benzène sulfonates, les méthane sulfonates, les iséthionates, les nicotinates, les isonicotinates, les embonates et les glucose phosphates.

Lorsque $R^3$ et $R^4$ forment ensemble le reste alcoylène d'un hétérocycle azoté, ils forment avec l'atome d'azote auxquels ils sont reliés, un reste pyrolidinyl, pipéridinyl, hexaméthylène imino ou lorsqu'ils incluent en outre un autre hétéroatome, un reste morpholinyl, tétrahydrothiazinyl, hexahydropyrimidinyl, hexahydropyrazinyl, pyrazolidinyl ou imidazolidinyl.

Un radical acyle est dérivé d'un acide organique caraboxylique ayant de 1 à 12 atomes de carbone, comme par exemple un acide alcoyl carboxylique, un acide arylcarboxylique, un acide arylalcoyl carboxylique, un acide cycloalcoyl carboxylique, un acide hétéroaryl carboxylique. On pourra citer à cet égard un acétyle, un butyryle, un benzoyle, un 3, 4 5-triméthoxybenzoyle, un cyclopropylcarbonyle ou un nicotinoyle.

Pour autant que l'invention soit concernée, un radical alcoyle inférieur est une chaîne hydrocarbonée ayant de 1 à 6 atomes de carbone, en chaîne droite ou ramifiée, comme par exemple le méthyle, l'éthyle, l'isopropyle, le secbutyle, le terbutyle, le pentyle, le néopentyle et le n-hexyle.

Un radical alcoxy inférieur a de 1 à 6 atomes de carbone dans le chaîne alcoyle qui peut être droite ou ramifiée comme un méthoxy, un éthoxy, un isopropoxy, un terbutoxy ou un pentyloxy.

La signification de paramètres n et n' est importante et joue un rôle significatif dans les propriétés pharmacologiques des composés de formule générale I. L'intensité ou la durée d'action des composés, selon l'invention, peut être modulée en modifiant la longueur de la chaîne carbonée de l'une ou l'autre partie de la molécule.

Parmi les composés de l'invention on citera plus particulièrement:
— la 1-[(4- p. fluorophényl 4-oxobutyl) -1] 4-[(N-carboxamido N' - méthyl guanidinyl) méthyl] pipéridine
— la 1-[(4-p. fluorophényl 4- oxobutyl) -1] 4-[(N-carboxamido N' - allyl guanidinyl) méthyl] piperidine
— la 1-[)4- p. fluorophényl 4- oxobutyl) -1] 4-[N-carboxamido N'- cyclo-propyl guanidinyl) méthyl] pipéridine
— la 1-[(4- p. fluorophényl 4-oxobutyl) -1] 4-[N-carboxamido N- cyclohexyl guanidinyl) methyl) pipéridine
— la 1-[(4- p. fluorophényl 4-oxobutyl) 1] 4-(N-carboxamido N'-morpholinyl) méthyl pipéridine son fumarate.

Les composés, selon l'invention, se distinguent par leurs propriétés pharmacologiques intéressantes et notamment par leurs propriétés anti-hypertensives. Ils manifestent à hautes doses un léger effet dépresseur sur le système nerveux central. Ils ne sont pas vasodilatateurs périphériques. Il n'influencent pas la diurèse. Du fait de leur haut niveau d'activité, les composés de formule générale I ou leurs sels d'addition trouvent un emploi en thérapeutique humaine ou animale comme principes actifs de médicaments destinés à combattre ou à réduire les effets de la maladie hypertensive, ou à traiter des affections vasculaires périphériques au cérébrales.

La demande de brevet britannique 2 034 305 décrit des dérivés de la pipéridine possédant une activité anti-hypertensive.

A ces fins, ils sont utilisés sous forme de compositions pharmaceutiques destinées à l'administration par voie parentérale, buccale, rectale ou sublinguale.

Les compositions pharmaceutiques renfermant, comme principe actif, au moins un composé de formule générale I ou un de ses sels d'addition avec un acide minéral ou organique en association ou en mélange avec un excipient ou un véhicule inerte pharmaceutiquement acceptable.

On pourra citer comme formes d'administrations préférées les comprimés nus ou enrobés, les capsules, les gélules, les dragées, les comprimés à noyaux multiples, les gouttes les solutions ou suspensions buvables; les solutés ou suspensions injectables réparties en ampoules, en flacons multi-doses ou en seringues auto-injectables; les suppositoires; les comprimés sublinguaux.

Les compositions pharmaceutiques, selon l'invention, peuvent, en outre, renfermer un ou d'autres principes actifs d'action similaire, complémentaire ou synergique. On pourra ainsi ajouter un diurétique du type thiazidique ou du type triamino-ptéridine; ou un agent beta bloquant comme le propanolol, le pindolol ou l'aténolol.

La posologie journalière peut vatier entre des limites larges en fonction de l'indication thérapeutique, de la voie d'administration, du malade et de l'ancienneté de la maldie hypertensive. En règle générale, chez l'adulte la posologie s'échelonne entre 0,1 et 50 mg par prise et entre 0,1 et 150 mg par jour.

D'une manière préférée, les compositions pharmaceutiques, selon l'invention, renferment entre 0,1 et 20 mg par prise unitaire.

Les composés de formule générale I peuvent être préparés par un procédé qui consiste à soumettre un cétal de (4-phényloxoalcoyl) pipéridine de formule générale II

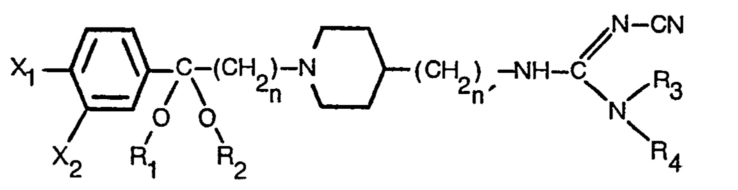

(II)

dans laquelle les substituants $X_1$, $X_2$, $R_3$, $R_4$, n et n' ont les signifactions fournies antérieurement.

$R_1$ et $R_2$ sont des radicaux alcoyle en $C_1$—$C_6$ ou forment ensemble une chaîne alcoylène inférieur à l'action d'un acide fort en milieu aqueux pour obtenir la carboxamidoguanidine de formule générale I que l'on peut si désiré, lorsque $R_4$ est de l'hydrogène, acyler par action d'un dérivé fonctionnel d'acide carboxylique pour former une N' - acyl N-carboxamido-guanidine de formule générale I.

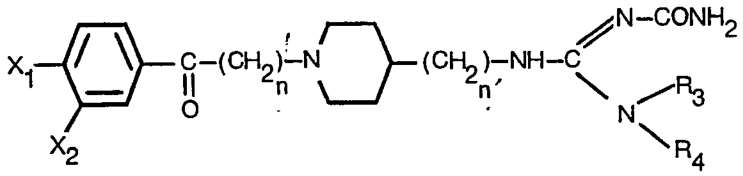

(I)

dans laquelle $X^1$, $X^2$, $R^3$, n et n' ont les définitions antérieures et $R^4$ le reste acyle d'un acide carboxylique ayant de 1 à 12 atomes de carbone, ou bien encore dédoubler par action d'un acide organique optiquement — actif en ses isomères optiques ou bien encore salifier par addition d'un acide minéral ou organique.

D'une manière préférée, l'acide fort est un acide minéral et notamment un acide halohydrique — comme l'acide chlorhydrique ou bromhydrique — ou l'acide sulfurique.

La réaction d'hydrolyse s'effectue de préférence en chauffant et en particulier à reflux du milieu réactionnel. Toutefois, des températures de réaction plus basses sont également possibles. Seul le temps de la réaction se trouve modifié.

Cette réaction d'hydrolyse simultanée de la fonction cétal en cétone et de la fonction nitrile en carboxamide demande un temps de chauffage bref et en général des temps de chauffage compris entre 1 minute et 60 minutes conviennent parfaitement.

Il est toutefois possible d'effectuer ces deux réactions en deux temps. On peut ainsi libérer la fonction cétone dans un premier temps par échange de fonction avec un acide cétonique ou un acide aldéhydique puis hydrolyser ensuite la fonction nitrile en carboxamide par action d'un acide fort.

La libération de la fonction cétone peut se faire commodément par action de l'acide glyoxylique ou de l'acide pyruvique.

L'hydrolyse de la fonction nitrile s'effectue par action de l'acide sulfurique ou chlorhydrique.

La salification des composés de formule générale I se fait commodément par action d'un acide minéral ou organique de préférence thérapeutiquement compatible en milieu aqueux, ou éthanolique, ou acétonique. Les sels ainsi obtenus sont généralement assez solubles dans l'eau et notamment les chlorhydrates.

Les sels peu solubles dans l'eau servent de moyens d'identification ou de purification.

Le dédoublement des composés de formule générale I s'effectue par salification à l'aide d'un acide optiquement actif comme par exemple l'acide d-tartrique, l'acide di O-benzoyltartrique, l'acide di O-toluyltartrique, l'acide d-camphosulfonique, l'acide d-camphorique, l'acide abietique ou l'acide pimarique. Les sels dédoublés ainsi formés sont ensuite convertis en base optiquement — active par action d'une base minérale ou organique.

Les composés de départ de formule générale II sont obtenus par un procédé qui consiste en ce que l'on fait réagir une 4- amino-pipéridine de formule générale III

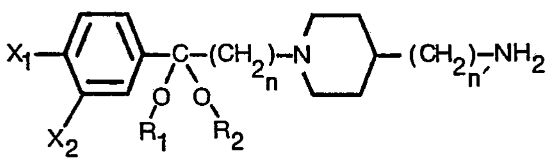

(III)

dans laquelle les substituants $X_1$, $X_2$, $R_2$, $R_2$, n et n' ont les significations fournies antérieurement avec un réactif de cyano imination choisi dans le groupe constitué parmi les cyano-imino isodithiocarbonate d'alcoyle de formule générale IV

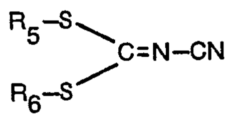

(IV)

dans laquelle $R_5$ et $R_6$ sont des radicaux alcoyle inférieur et les cyanoimino isothiocarbonates mixtes d'alcoyle de formule générale V

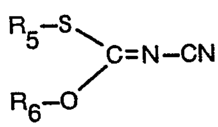

(V)

dans laquelle $R_5$ et $R_6$ représentent dex radicaux alcoyle inférieur pour former une isothiourée ou une isourée de formule générale VI

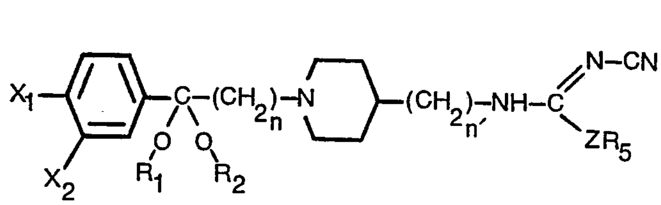

dans laquelle $X_1$, $X_2$, $R_1$, $R_2$, $R_5$, Z, n et n' sont définis comme précédemment que l'on fait réagir avec une amine primaire ou secondaire de formule

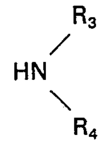

dans laquelle $R_3$ possède les significations antérieures et $R_4$ représente de l'hydrogène, un radical alcoyle en $C_1$—$C_6$ inférieur, ou forment ensemble le reste alcoylène d'un hétérocycle azoté pour former une cyano-guanidine de formule générale II

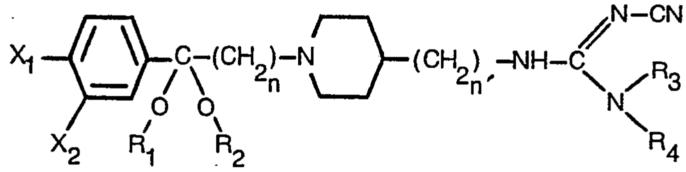

(II)

dans laquelle $X_1$, $X_2$, $R_1$, $R_2$, $R_3$, n et n' sont définis comme précédemment et $R_4$ est de l'hydrogène ou un reste alcoyle en $C_1$—$C_6$.

Les exemples suivants illustrent l'invention sans toutefois la limiter.

Exemple I

1-[4-p. fluorophényl 4-oxo)-1 butyl] 4-[(N-carboxamido N'-méthyl) guanidinylméthyl] pipéridine et son fumarate

On porte ½ heure à ébullition, 9,5 grammes de [(4-fluorophényl 4-éthylènedioxy) - 1 butyl]-1 [(N-cyano n' - méthyl) guanidinylméthyl] -4 pipéridine et 100 ml d'acide chlorhydrique en solution normale.

On refroidit, rend alcalin par de la soude, extrait à l'acétate d'éthyle, sèche et concentre à sec la solution organique.

On obtient 3,3 grammes d'un produit visqueux semi-solide qui est transformé en fumarate cristallisé par addition d'une solution de 1,1 grammes d'acide fumarique dans l'éthanol. Le fumarate précipite rapidement. On le sépare par filtration et essorage. On obtient 3,4 grammes de cristaux Fi = 220°.

Le fumarate de 1- (p. fluorophényl 4-oxobutyl-1) 4-[(N-carboxamido N'- méthyl) guanidinyl méthyl] pipéridine est soluble dans l'eau mais peu soluble dans les solvants organiques.

Exemple II

1-[(4- p. fluorophényl 4-oxo)-1 butyl] 4[(N- carboxamido N'- cyclopropyl) guanidinylméthyl]pipéridine et son fumarate

On porte à l'ébullition une suspension de 5 grammes de 1-[(4- p. fluorophényl 4- éthylènedioxy) - 1 butyl] 4[(N- cyano N'-cyclopropyl) guanidinyl méthyl]pipéridine 2 minutes à l'ébullition avec 100 ml d'une solution d'acide chlorhydrique normale.

Après refroidissement, on rend alcalin par de la soude et épuise la solution aqueuse par du chloroforme. Après évaporation à sec du solvant, on obtient 3,7 grammes d'une huile épaisse qui est transformée en fumarate par ébullition 5 minutes avec une solution de 1 gramme d'acide fumarique dans 50 ml d'éthanol.

On obtient 3,5 grammes du fumarate cherché sous forme de cristaux blancs de Fi = 190°.

Exemple III

1-[(4- p. fluorophényl 4-oxo) - 1 butyl] 4-[N-carboxamido N'-allyl)guanidinylméthyl] - pipéridine et son fumarate

6 grammes de 1-[(4- p. fluorophényl 4,4 éthylènedioxy) butyl]4-[N- cyano N'-allyl) guanidinylméthyl]pipéridine sont mis en solution dans 300 ml d'acide sulfurique normal.

Après un repos d'une semaine à température ambiante, la solution est rendue alcaline par addition de soude et extraite au chloroforme. Les phases chloroformiques sont séparées, lavées à l'eau acide puis à l'eau, séchées et concentrées à sec. On obtient 5,7 g d'un produit huileux que l'on convertit en fumarate au moyen de 1,6 grammes d'acide fumarique dans l'éthanol.

Les cristaux formés sont séparés pa filtration, lavés et séchés. On obtient 6,1 g du fumarate du produit cherché, qui cristallise avec une demi-molécule d'eau Fi = 190—2°.

Exemple IV

1-[(4- p. fluorophényl 4- oxo) butyl - 1] 4-[(N-carboxamido N'-cyclohexyl) guanidinylméthyl]pipéridine

*Stade A*

1-[(4- p. fluorophényl 4- oxo) butyl - 1] 4-[(N-cyano N' - cyclohexyl) guanidinylméthyl]pipéridine

On porte à l'ébullition 5 grammes de 1- (4- p. fluorophényl 4,4 éthylènedioxy) butyl]4-[N-cyano S-

méthylisothiouréïdo) - méthyl]pipéridine, 20 ml de pyridine et 30 ml de cyclohexylamine. La réaction est suivie par chromatographie en couche mince et après 5 heures, la réaction est totale.

On concentre alors à sec le milieu réactionnel et l'huile obtenue est traitée par l'éther isopropylique. Le solide formé est filtré, lavé et séché à sec puis recristallisé dans l'acétonitrile.

Ob obtient 3,6 g de 1-[(4- p. fluorophényl 4,4-éthylènedioxy) butyl]4-[N- cyano N'- cyclohexyl guanidinométhyl)pipéridine de PF = 131—2°.

Pour hydrolyser le cétal, on met ce produit en solution dans 150 ml d'acide chlorhydrique et 100 ml d'éthanol. Après quelques heures de repos à la température ambiante, il se dépose de beaux cristaux incolores. Le chlorhydrate du produit cherché ainsi formé est séparé par filtration, lavé et séché. On recueille 3 grammes de produit de Fi = 170°.

La base est libérée par traitement à la soude et extraction à l'acétate d'éthyle.

On obtient ainsi un produit visqueux qui cristallise par reprise avec de l'acétonitrile. Le dérivé cyclohexylé est séparé par filtration, lavé à l'acétonitrile et séché sous vide.

On récupère de cette façon le produit cherché sous forme de cristaux blancs fondant à 145—146°.

*Stade B*

1-[(4- p. fluorophényl 4- oxo) butyl - 1] 4-[N- carboxamido N' - cyclohexyl) guanidinylméthyl] pipéridine et son fumarate

on met en suspension 2g10 de 1-[(4- p. fluorophényl 4- oxo) butyl - 1] 4-(N-cyano N' - cyclohexyl guanidinyl) méthyl] pipéridine dans 50 ml d'une solution normale d'acide chlorhydrique.

On porte le mélange au reflux pendant 30 minutes, puis on laisse revenir à température ordinaire.

On ajoute de la soude jusqu'à réaction franchement alcaline du milieu. On épuise alors au chlorure de méthylène à trois reprises. On réunit les phases organiques, on les lave à l'eau, on les sèche et on les filtre. On amène ensuite à sec par distillation sous pression réduite.

On récupere ainsi un résidu huileux pesant 1g21. On le transforme en fumarate par reprise dans le minimum d'éthanol chaud et addition d'une solution de 1g1 d'acide fumarique dans 25 ml d'éthanol. On amorce la cristallisation par grattage puis laisse reposer 12 heures en chambre froide. On sépare alors les cristaux par filtration, les essore, les rince avec quelques ml d'éthanol puis les sèche sous vide.

Le fumarate récupéré se présente suis forme de cristaux incolores fonaant à 136—137°.

Exemple V

1-[(4- p.fluorophényl 4- oxo) butyl - 1] 4-[N- carboxamido N' - morpholyl) guanidinylméthyl] pipéridine

*Stade A*

1-[(4- p. fluorophényl 4-oxo) butyl]4-[(N-cyano N'-morpholyl) guanidinylméthyl]pipéridine

On porte 16 heures à l'ébullition, un mélange de 1-[(4- p. fluorophényl 4,4 - éthylènedioxy) butyl]-4-[(N-cyano S- méthylisothiouréïdo) - méthyl]pipéridine et de 100 ml de morpholine.

Après concentration à sec, on obtient 9 grammes d'huile incristallisable, qui sont mis en solution dans 250 ml d'acide chlorhydrique normal et ce mélange est laissé au repos 17 heures à température ordinaire.

Après alcalinisation du milieu par addition de soude puis extraction à l'acétate d'éthyle de la phase aqueuse, on obtient aprés amenée à sec 4 grammes d'huile qui est purifiée par chromatographie sur silice H Merk en employant le mélange $CHCL_3$ - isopropylamine(9:1) comme éluant.

On obtient 3 grammes de produit sous forme d'huile qui cristallise alors par reprise à l'éther isopropylique. La 1-[(4- p. fluorophényl 4-oxo) butyl -1] 4-(N-cyano N'- morpholyl) guanidinylméthyl]pipéridine se présente sous forme de cristaux incolores fondant à 121—122°.

*Stade B*

1-[(4- p. fluorophényl 4-oxo) butyl]- 1] 4-[N- carboxamido N'-morpholyl) guanidinylméthyl]pipéridine

On dissout 2g4 de 1-[(4- p. fluorophényl 4-oxo) butyl]- 4-[(N-cyano N'-morpholyl) guanidinyl méthyl]pipéridine obtenue au stade A du présent exemple dans 35 ml de Dioxane et on y ajoute 15 ml d'acide chlorhydrique 5N. On porte le mélange au reflux du solvant pendant 45 mn. On laisse ensuite refroidir à température ordinaire, on alcalinise franchement le milieu réactionnel par addition progressive de lessive de soude, puis on épuise la phase aqueuse au chlorure de méthylène. Les phases méthyléniques sont réunies, lavées à l'eau séchées et évaporées à sec.

Le résidu visqueux est repris par de l'éther isopropylique à chaud. Par refroidissement la carboxamido-guanidine précipite.

On sépare les cristaux qu'on essore, lave à l'éther isopropylique puis sèche sous vide.

On obtient ainsi 1g05 de carboxamide-guanidine sous forme de cristaux incolores fondant à 105—106°. Le fumarate fond à 170°.

Exemple VI

En opérant de la même manière au départ de 1-(4- p.fluorophényl 4,4 éthylènedioxy butyl-1) 4- amino pipéridine on obtient:

— la 1-(4-p. fluorophényl 4-oxobutyl-1) 4-(N-carboxamido N'-allylguanidinyl) pipéridine sous forme de fumarate F = 186° (le spectre Infra-Rouge et la micro-analyse sont conformes avec la structure annoncée).

— la 1-(4-p. fluorophényl 4-oxobutyl-1) 4-(N-carboxamido N'-méthyl guanidinyl) pipéridine sous forme de fumarate cristallisé avec molécule d'eau F = 195° environ. La microanalyse et le spectre Infra-Rouge sont conformes à la structure annoncée.

Au départ de la (1-phényl 4,4-éthylènedioxy butyl-1) 4-aminopipéridine on a préparé la 1-(4-phényl 4-oxobutyl-1) 4-(N-carboxamido N'-cyclopropylguanidinyl) pipéridine isolée sous forme de chlorhydrate F = 210—211°.

### Exemple VII

Réalisation de comprimés à 5 mg de principe actif.
Fumarate de 1-[(4- p. fluorophényl 4-oxo) butyl - 1] 4-[(N-carboxamido N'-méthyl) guanidinyl méthyl]pipéridine 65,1 g

(correspondant à 50 g de base)

| | |
|---|---|
| Amidon de blé | 625 g |
| Amidon de maïs | 545 g |
| Cellulose micro-cristalline | 46 g |
| Carboxyméthyl amidon | 17 g 5 |
| Méthylcellulose | 4 g 5 |
| Stéarate de magnésium | 22 g |
| Talc | 22 g 5 |

pour 10,000 comprimés terminés au poids moyen de 0g150

### Example VIII

Etude pharmacologique des composés selon l'invention.

a) *Détermination de la toxicité aiguë*

Une dose léthale moyenne ($DL_{50}$) approchée a été déterminée après administration par voie orale des composés, selon l'invention, à doses croissantes à des lots de 10 Souris femelles EPOS d'élevage CESAL, par la méthode de D.E.J. Campbell et W. Richter (Acta Pharmacol. and Toxicol. *25* (1967) 345).

Les animaux ont été gardés en observation pendant 5 jours. Les morts quand il y en a sont dénombrés. Les doses léthales moyennes sont de l'ordre de 1200 mg/kg.

b) *Recherche d'un effet sur le système nerveux central*

Aux doses élevées (60 mg/kg) on constate chez les Souris une hypothermie importante, une ptose des paupières, une diminution de la matricité et des réactions d'éveil.

c) *Détermination de l'effet anti-hypertensif*

L'essai a été réalisé sur des lots de rats mâles vigiles rendus hypertendus par ligature de l'aorte abdominale.

Les produits, selon l'invention, ont été administrés par voie orale aux doses de 2,5 ou 10 mg/kg. Ils entraînent une baisse de pression nette et prolongée.

Par ailleurs, ces mêmes composés entraînent en outre une très nette hypotension pour des doses de 100 et 500 µg/kg lorsqu'ils sont administrés par voie intraveineuse à des rats normotendus ou à des chiens normotendus anesthésiés.

d) *Recherche d'un effet vasodilatateur*

Les composés, selon l'invention, ne provoquent pas d'effet vasodilatateur périphérique. On ne constate chez le rat, particulièrement au niveau des pattes postérieures, aucune vasodilatation ni d'augmentation de la température cutanée. Cet effet ne se manifeste pas même pour des doses allant jusqu'à 10 mg/kg.

**Revendications**

1. Les (phényloxoalcoyl) pipéridinoguanidines de formule générale

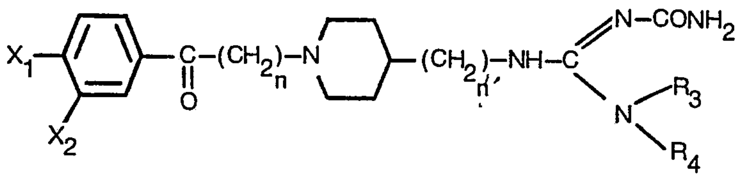

(I)

dans laquelle $X_1$ et $X_2$, identiques ou différents, représentent de l'hydrogène, un radical alcoyle en $C_1$—$C_6$, un radical alcoxy en $C_1$—$C_6$, un halogène ou un radical trifluorométhyle, $R_3$ représente un radical alcoyle en $C_1$—$C_6$, $R_4$ représente de l'hydrogène, un radical alcoyle en $C_1$—$C_6$ ou le reste acyle d'un acide organique carboxylique ayant de 1 à 12 atomes de carbone, ou bien $R_3$ et $R_4$ forment ensemble le rest alcoylène d'un hétérocycle azoté choisi dans le groupe constitué par un radical pyrolidinyl, pipéridinyl, hexaméthylène imino, morpholyl, tétrahydrothiazinyl, hexahydropyrimidinyl, hexahydropyrazinyl, pyrazolidinyl, ou imidazolidinyl,

n est égal à 1, 2 ou 3
n' est égal à 0 ou 1

ainsi que les composés suivants: 1-[(4.p-fluorophenyl 4-oxo)1-butyl]4-[(N-carboxamido N'-allyl) guanidinylmethyl]piperidine, 1-[4-(p fluorophenyl 4-oxo)butyl-1] 4[-(N-carboxamido N'-cydohexyl) guanidinylmethyl]piperidine and 1-[(4-fluorophenyl 4-oxo)butyl-1] 4-(N-Carboxamido N'-allyl-guanidinyl)piperidine sous forme racémique ou sous forme optiquement — active.

2. Les sels d'addition des composés selon la revendication 1 avec un acide minéral ou organique.

3. Un composé selon une des revendications 1 ou 2 à savoir la 1-[(4- p. fluorophényl 4-oxo) butyl-1] 4-[(N-carboxamido N' - méthyl) guanidinylméthyl] pipéridine et son fumarate.

4. Un composé selon l'une des revendications 1 ou 2 à savoir la 1-[(4-p. fluorophényl 4-oxo) butyl - 1] 4-[(N-carboxamido N' - cyclopropyl) guanidinylméthyl] pipéridine et son fumarate.

5. Un composé selon l'une des revendications 1 ou 2 à savoir la 1-[(4-p. fluorophényl 4-oxo) butyl - 1] 4-[(N-carboxamido N' - allyl) guanidinylméthyl] pipéridine et son fumarate.

6. Un composé selon l'une des revendications 1 ou 2 à savoir la 1-[(4-p. fluorophényl 4-oxo) butyl - 1] 4-[(N-carboxamido N' - cyclohexyl)] guanidinylméthyl] pipéridine et son fumarate.

7. Un composé selon la revendication 1 à savoir la 1-[(4-p. fluorphényl 4-oxo) butyl- 1] 4-[(N-carboxamido N' - morpholyl) guanidinylméthyl] pipéridine.

8. Les compositions pharmaceutiques renfermant à titre de principe actif au moins un composé selon la revendication 1 ou un de leurs sels pharmaceutiquement acceptables en association ou en mélange avec un excipient ou un véhicule inerte.

9. Les compositions pharmaceutiques, selon la revendication 8, dans lesquelles le véhicule ou l'excipient inerte est un de ceux adaptés pour l'administration par voie buccale, parentérale, rectale, percutanée ou sublinguale.

10. Les compositions pharmaceutiques selon l'une des revendications 8 ou 9 qui renferment en outre un ou d'autres principes actifs d'action similaire, complémentaire ou synergique.

11. Les compositions pharmaceutiques, selon l'une des revendications 8 à 10 dans lesquelles la teneur en principe actif, selon la revendication 1, s'échelonne de 0,1 à 20 mg par prise unitaire.

12. Un procédé d'obtention des composés de formule générale I, selon la revendication 1

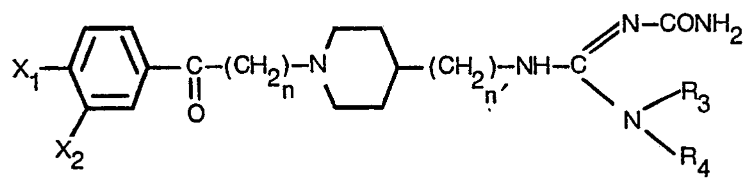

(I)

dans laquelle les substituants X1, X2, R3, R4, n et n' gardent les significations antérieures caractérisé en ce que l'on soumet un cétal de (4- phényl-oxoalcoyl) pipéridine de formule générale II

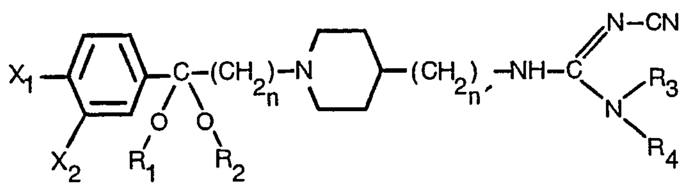

(II)

dans laquelle le substituants X1, X2, R3, R4, n et n' ont les significations fournies antérieurement.

$R_1$ et $R_2$ sont des radicaux alcoyle inférieur ou forment ensemble une chaîne alcoylène inférieur à l'action d'un acide fort en mileiu aqueux pour obtenir la carboxamidoguanidine et formule générale I que l'on peut si désiré lorsque $R_4$ est de l'hydrogène, acyler par action d'un dérivé fonctionnel d'acide carboxylique pour former une N' - acyl N - carboxamidoguanidine de formule générale I

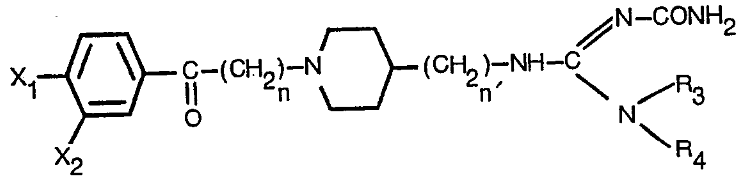

(I)

dans laquelle $X_1$, $X_2$, $R_3$, n et n' ont les définitions antérieures, et $R_4$ est le reste acyle d'un acide carboxylique ayant de 1 à 12 atomes de Carbone, ou bien encore dédoubler par action d'un acide organique optiquement — active en ses isomères optiques, ou bien encore salifier par addition d'un acide minéral ou organique.

**Patentansprüche**

1. Die (Phenyloxy alkyl) piperidino guanidine der allgemeinen Formeln I

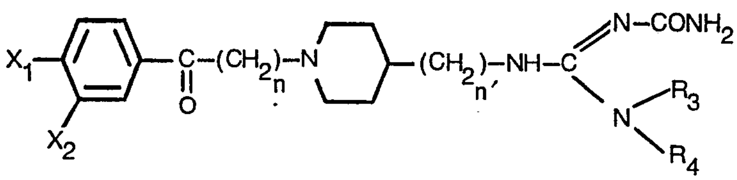

(I)

worin $X_1$ und $X_2$, gleich or verschiedene, fur ein Wasserstoff, eine $C_1$—$C_6$ Alkyl gruppe, eine $C_1$—$C_6$ Alkoxy gruppe, ein Halogen, oder eine Trifluoromethyl gruppe stehen $R_3$ eine $C_1$—$C_6$ Alkyl gruppe bedeutet, $R_4$ ein Wasserstoff, eine $C_1$—$C_6$ Alkyl gruppe oder ein von einer organischen Carbonsäure mit 1 bis 12 Kohlenwasserstoff Atome, ableitende Acylrest bedeutet, oder $R_3$ und $R_4$ zusammen den Alkylene Rest eines Nitrogen-haltenden Heteroring aus der Gruppe von pyrolidinyl, piperidinyl, hexamethylene imino, morpholyl, tetrahydrothiazinyl, hexahydropyrimidinyl, hexahydropiperazinyl, pyrazolidinyl und imidazolidinyl Rest gewühlt ist.

n gleich 1, 2 oder 3.
n' gleich 0 oder 1 ist.

sowie als die folgende Verbindungen: 1-[(4-p.fluorphenyl 4-oxo)-1 butyl] 4-[(N-carboxamido N'-methyl) guanidinyl methyl] piperidin, 1-[4-(p.fluorphenyl 4-oxo)-1 butyl] 4-[(N-carboxamido N'-cyclohexyl) guanidinyl methyl] piperidin und 1-[(4-p. fluorophenyl 4-oxo)-1 butyl] 4-(N-carboxamido N'-allyl-guanidinyl)piperidin.

2. Die Zusatzsalze der Verbindungen nach Anspruch 1, mit einer anorganischen oder organischen Saüre.

3. Eine Verbindung nach einer der Ansprüche 1 oder 2, d.h. 1-[(4-p.fluorophenyl 4-oxo) butyl-1] 4-[(N-carboxamido N'-methyl) guanidinyl methyl] piperidin und sein Fumarat.

4. Eine Verbindung nach einer der Ansprüche 1 oder 2, d.h. 1-[(4-p. Fluorphenyl 4-oxo) butyl-1] 4-[(N-carboxamido N'-cyclopropyl) guanidinylmethyl] piperidin und sein Fumarat.

5. Eine Verbindung nach eine der Ansprüche 1 oder 2, d.h. 1-[(4-p.Fluor phenyl 4-oxo) butyl-1] 4-[(N-carboxamido N'-allyl) guanidinylmethyl] piperidin und sein Fumarat.

6. Eine Verbindung nach einer der Ansprüche 1 oder 2, d.h. 1-[(4-p. Fluorphenyl 4-oxo) butyl-1] 4-[(N-carboxamido N'-cyclohexyl) guanidinylmethyl] piperidin und sein Fumarat.

7. Eine Verbindung nach einer der Ansprüche 1 oder 2, d.h. 1-[(4-p. Fluorphenyl 4-oxo) butyl-1] 4-[(N-carboxamido N'-morpholyl) guanidinylmethyl] piperidin.

8. Die pharmazeutische Zubereitungen, die als Wirkstoff mindenstens eine Verbindung nach Anspruch 1 oder einer pharmazeutische-vertrüglichen Salz, enthalten in Verbindung oder Vermischung mit einem inerten Träger oder Vehikel.

9. Die pharmazeutische Zubereitungen nach Anspruch 8, worin der Träger oder Vehikel ein der für orale, parenterale, rektale, percutan oder sublingual Verabreichung geignet ist.

10. Die pharmazeutische Zubereitungen nach einer der Ansprüche 8 oder 9 die darüber eine oder mehre Wirkstoffe mit einer gleiche, zusätzliche oder synergistiche Wirkung enthalten.

11. Die pharmazeutische Zubereitungen nach einer der Ansprüche 8 bis 10 worin das Gehalt an Wirkstoff nach Anspruch 1 zwischen 0,1 und 20 mg per einzelnem Dosis liegt.

12. Verfahren zur Bereitung der Verbindungen oder allgemeinen Formel I, nach Anspruch 1

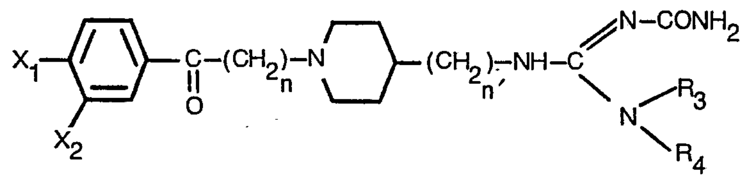

(I)

worin die substituenten $X_1$, $X_2$, $R_3$, $R_4$, n und n' die vorher angegebene Bedeutungen besitzen, dadurch gekennzeichnet daß ein Ketal von (4-phenyl oxo alkyl) piperidin der allgemeinen Formel II

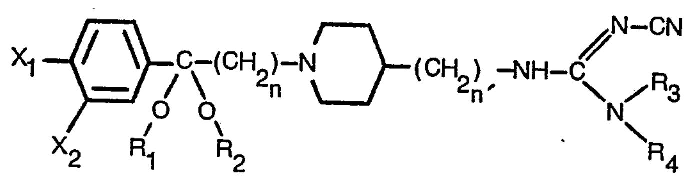

(II)

worin die substituenten $X_1$, $X_2$, $R_3$, $R_4$, n und n' die vorher angegebene Bedeutungen haben.

$R_1$ und $R_2$ ein niedrig-molekular Alkylrest sind oder zusammen eine niedrig Alkylene Kette bilden, die Wirkung einer starken Saüre in wässrige Medium unterwirft um das Carboxamidoguanidin der allgemeinen Formel I zu gewinnen, das, wenn gewünscht, wenn $R_4$ ein Wasserstoff ist, mittels einem funktionellen Derivate einer Carbonsäure acylieren kann, um ein N'-acyl N-Carboxamidoguanidin der allgemeinen Formel I

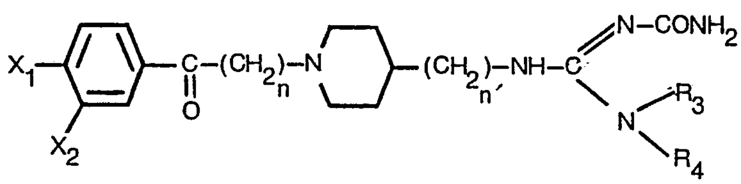

(I)

worin $X_1$, $X_2$, $R_3$, n und n' die vorherige Bedeutungen haben, zu gewinnen, und $R_4$, den Acylrest einer Carbonsaüre mit 1 bis 12 Kohlenstoff Atome ist oder noch in seiner optischen Isomeren mittels einer optische-aktiven organischen Saüre spalten kann, oder noch beim Zusatz einer anorganischen oder organischen Saüre in einem Salz überfuhrt.

**Claims**

1. The (phenyloxyalkyl) piperidinoguanidines of the formula:

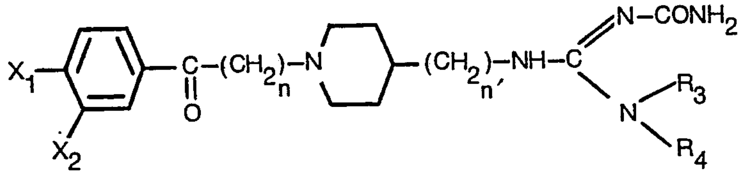

(I)

wherein $X_1$ and $X_2$, the same or different, are a hydrogen, a $C_1$—$C_6$ alkyl group, a $C_1$—$C_6$ alkoxy group, a

halogen, a trifluoromethyl group. $R_3$ is a $C_1$—$C_6$ alkyl group. $R_4$ is a hydrogen, a $C_1$—$C_6$ alkyl group, or the acyl residue of an organic carboxylic acid having from 1 to 12 carbon atoms, or $R_3$ and $R_4$ together, are the alkylene group of a nitrogenous heterocycle selected from the group consisting of a pyrrolidinyl, a piperidinyl, a hexamethylene imino, a morpholyl, a tetrahydrothiazinyl, a hexahydropyrimidyl, a hexahydropyrazinyl, a pyrazolidinyl or an imidazolidinyl group.

n is equal to 1, 2 or 3.
n' is equal to 0 or 1,

as well as the following compounds: 1-[(4.p-fluorophenyl 4-oxo)-1-butyl]4-[(N-carboxamido N'-allyl) guanidinyl methyl]piperidine, 1-[(4-(2 fluorophenyl 4-oxo)butyl-1]4[-(N-carboxamido N'-cydohexyl) guanidinyl methyl]piperidine and 1-[(4-p.fluorophenyl 4-oxo)butyl-1] 4-(N-Carboxamido N'-allyl guanidinyl) piperidine, in the racemic or optically-active form.

2. The acid addition salts of the compounds according to claim 1, with a mineral or organic acid.

3. A compound according to claim 1 or claim 2, i.e. 1-[(4-p.fluorophenyl 4-oxo) butyl-1]4-[(N-carboxamido N'-methyl) guanidinylmethyl]piperidine and its fumaric acid addition salt.

4. A compound according to claim 1 or claim 2 i.e. 1-[(4-p.fluorophenyl 4-oxo) butyl-1] 4-[(N-carboxamido N'-cyclopropyl) guanidinylmethyl]piperidine and its fumaric acid addition salt.

5. A compound according to claim 1 or claim 2 i.e. 1-[(4-p.fluorophenyl 4-oxo) butyl-1]4-[(N-carboxamido N'-allyl) guanidinyl methyl]piperidine and its fumaric acid addition salt.

6. A compound according to claim 1 or claim 2 i.e. 1-[(4-p.fluorophenyl 4-oxo) butyl-1]4-[(N-carboxamido N'-cyclohexyl)guanidinyl methyl]piperidine and its fumaric acid addition salt.

7. A compound according to claim 1 i.e. 1-[(4-p.fluorophenyl 4-oxo) butyl-1]4-(N-carboxamido N'-morpholyl) guanidinyl methyl]piperidine.

8. The pharmaceutical compositions containing as active ingredient at least one compound according to claim 1 or a pharmaceutically-acceptable salt thereof, in conjunction or admixture with an inert carrier or vehicle.

9. The pharmaceutical compositions according to claim 8 wherein the inert carrier or vehicle is one of those suitable, for administration through the oral, parenteral, rectal, percutaneous or sublingual ways of administration.

10. The pharmaceutical compositions according to any of the claims 8 to 9 which further contain one or more active ingredients of similar, complementary or synergistic activity.

11. The pharmaceutical compositions according to claims 8 to 10 wherein the content in active ingredient according to claim 1 ranges from 0,1 to 20 mg per unit dosage.

12. A process for producing the compounds of formula I according to claim 1

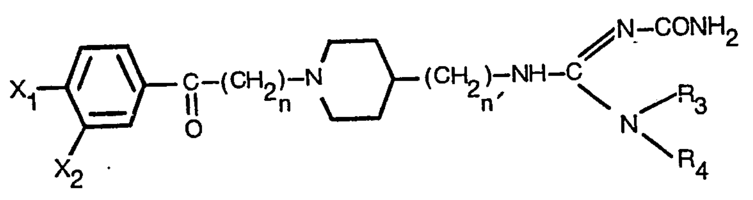

(I)

wherein the substituents $X_1$, $X_2$, $R_3$, $R_4$, n and n' retain the previously-given definitions, characterized in that a Ketal of (4-phenyl oxo butyl) piperidine of formula II

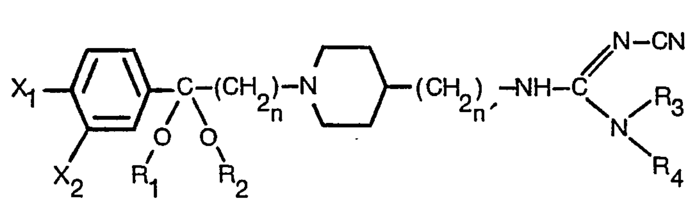

(II)

wherein the substituents $X_1$, $X_2$, $R_3$, $R_4$, n and n' have the previously-given definitions, $R_1$ and $R_2$ are lower alkyl radicals or together form a lower alkylene chain, is submitted to the action of a strong acid in an aqueous medium to produce a carboxamidoguanidine of formula I which can, if desired, when $R_4$ is hydrogen, be acylated by means of a functional derivative of a carboxylic acid, to produce a N'-acyl N-carboxamido guanidine of formula I

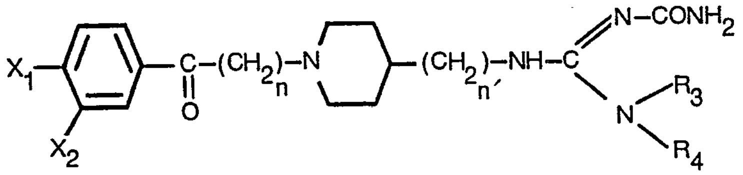

(I)

wherein $X_1$, $X_2$, $R_3$, n and n' have the previously-given definitions and $R_4$ is the acyl residue of an organic carboxylic acid having from 1 to 12 carbon atoms, or be resolved by means of an optically-active organic acid, into its optical isomers or be salified by adding a mineral or organic acid.